# EUROPEAN PATENT APPLICATION

(11) **EP 3 967 215 A1**
(43) Date of publication of application: **16.03.2022**
(21) Application number: 20196288.3
(22) Date of filing: 15.09.2020
(51) Int. Cl.: A61B 5/00, A61B 5/01, A61B 5/0507, A61B 5/024, A61B 5/08, A61B 5/11, A61B 5/1171

(54) **MEASUREMENT DEVICE AND METHOD FOR ASSESSING VITAL DATA OF A HUMAN OR ANIMAL SUBJECT**

(71) Applicant: Alunos AG, 6314 Unterägeri (CH)
(72) Inventor: PEYER, Christof, 7214 Grüsch (CH); WIRTH, Matthias, 7000 Chur (CH)
(74) Representative: Rutz, Andrea

(57) **Abstract**

A measurement device (10) for assessing vital data of a human or animal subject (1,35) is provided, comprising one or several thermal sensors (13) for the contactless measurement of temperature data in two spatially different regions (6,8) of the subject, wherein one of the two spatially different regions (6,8) is located in the nose-mouth area. The measurement device also comprises a processing device (14) which is configured to determine a vital value based on a difference in the temperature data between the two spatially different regions (6,8). A corresponding method for assessing vital data is also provided. Furthermore, a measurement device (39) and a method are provided, in which a region-of-interest (38) is identified based on data from a CMOS- and/or a time-of-flight-sensor (41,42), in order to determine a vital value based on measurement data obtained from a radar-emitter and -receiver (52,53).

## Description

### TECHNICAL FIELD

The present invention relates to a measurement device and to a method for assessing vital data of a human or animal subject. The measurement device and method can particularly be used for assessing the breathing and/or the core temperature of the subject.

### PRIOR ART

Problems with airways like congestions can be indicators for health issues of a subject to be examined. The detection of such problems without close personal contact by a health specialist or without direct contact by diagnostic equipment is advantageous. Contactless measurements have for example the advantage that the risk of infections being transmitted from the patient to the health specialist and vice versa is greatly reduced. In addition, contactless measurements usually are more comfortable for the patient. Measurements that do not require any physical contact usually also have less impact on the measured subject and, thus, have less influence on the vital property to be measured, such that the measurement results become more reliable.

One type of vital data that can be an important indicator for the state of health of a patient, are particularly data that concern the breathing behavior of the measured subject. Based on the combination of for example the information of whether the measured subject is mostly breathing through the mouth or through the nose with the breathing rate, the amplitude and the time course of the respiratory curve, conclusions can be drawn about the quality of respiration and the general state of health of a patient. Abnormal respiration patterns like for example Tachypnea, Bradypnea, Apnea, Cheyne-Stokes, Biot's, Air trapping or Kussmaul's can be detected. Based on the detected respiration pattern, not only a potential congestion of the airways can be detected, but it might even be possible to draw conclusions about which parts are affected. A congestion of airways can be an indicator for a particular disease. For example, certain illnesses affect the sinus cavities, while others mostly affect the lungs. A screening of the breathing behavior can also be used for the detection of COVID-19.

WO 2019/239124 A1 discloses an apparatus for determining respiratory information about a user during sleep. A CCD camera, an infrared camera and/or a thermal imaging camera are used to monitor a surface temperature of the user in the region of the mouth and the nose. The temperature curve of the exhaled air is assessed for this purpose.

US 2020/0138292 A1 is directed to the monitoring of the respiration by means of noncontact thermal imaging. The measured data are used to reconstruct 4D models of exhale episodes, in order to assess the breathing quality.

Thermal imaging in the region of mouth and nose, in order to determine respiratory characteristics of an animal, is disclosed by WO 2015/030611 A1.

DE 10 2012 216 013 A1 proposes the imaging of exhaled breath by means of a microwave-infrared camera. The obtained imaging data are used to determine the concentration of CO₂ in the exhaled breath.

Another important indicator for the state of health of a patient is the core temperature. Attempts were made to determine the core temperature of human or animal subjects without contact, which, however, mostly led to inaccurate and unreliable results. A common problem when measuring the core temperature of a subject without contact, e.g. by means of a thermal sensor, is the deviation of the skin temperature (normally measured in the face) and the core temperature of the subject. A wrong measurement result for the core temperature can particularly be obtained, if the ambient temperature of the measurement location or of the location where the measured subject has been located before the measurement significantly differs from the core temperature of the subject. For example, if a person enters a building after staying for a longer time outdoors on a cold winter day, the determination of the core temperature is often unreliable and inaccurate, when applying state-of-the-art methods with a thermal sensor for measuring the skin temperature, in order to draw conclusions concerning the core temperature.

US 2018/0085006 A1 and WO 2011/6774 A1 disclose methods in which the core temperature is determined based on a thermal image of the open mouth.

Furthermore, devices are known for the assessment of vital data, which are based on reflections of radar waves, in order to measure e.g. the movement of a person's chest. By measuring the propagation time of the reflected radar wave from the patient to the radar receiver, the relative or absolute distance between e.g. the surface of the person's chest and the radar receiver can be determined. Based on the measurement of the reflected radar waves, it is possible for example to determine the breathing behavior of a patient or the number of his or her heartbeats per minute. In many situations, however, the data obtained in such a radar-measurement are not reliable. For example, if a group of two or more subjects is present in the measurement zone of a radar sensor, difficulties might arise to assign the recorded signals to a single subject or to even recognize that more than a single person is present. Movements - intentional and unintentional - of the subject to be examined can also falsify the measurement results or can even render the measurement impossible.

CN 111191588 A discloses a method in which measured Doppler radar datasets are combined with CCD- or CMOS-images. The method is not used to assess vital data, but to improve behaviour recognition.

DE 20 2017 106 970 U1 discloses a device which combines data acquired by a millimeter-wave-radar device with time-of-flight(ToF)-data, in order to identify locations of humans/animals in the event of a catastrophe.

### SUMMARY OF THE INVENTION

It is an object of the present invention to enable an accurate and reliable assessment of vital data of a human or animal subject by means of a contactless measurement.

This object is solved by a first type of a measurement device as claimed in claim 1 and by a first method as claimed in claim 15. Further embodiments of the measurement device and the method are provided in the dependent claims.

The present invention thus provides a first type of measurement device for assessing vital data of a human or animal subject, comprising:
one or several thermal sensors for the contactless measurement of temperature data in at least two spatially different regions of the subject, preferably directly on the skin surface of the subject, wherein at least one of the at least two spatially different regions is located in the nose-mouth area of the subject; and
a processing device which is coupled to the one or several thermal sensors and which is configured to determine at least one vital value, which is characteristic of the subject, on the basis of a difference in the measured temperature data between the spatially different regions of the subject.

By determining the at least one vital value on the basis of a difference in the temperature data between two spatially different skin regions of the subject, a more accurate and reliable result can be achieved. Since the determination is based on a temperature difference between two spatially different skin regions, the result is less affected by external influences, such as in particular the ambient temperature. Since external influences usually affect the two spatially different regions likewise, the effect on the determined vital value can be reduced.

The at least one vital value can for example concern the respiration of the subject. It can, in this case, for example refer to the information, whether the subject mostly breathes through the nose or through the mouth, or reflect the amount of air that is inhaled or exhaled. The at least one vital value can be in the form of a single number or in the form of a time course of e.g. the amount of inhaled or exhaled air over one or more breathing cycles. The vital value can also be in the form of an indicator for a certain breathing pattern, which can particularly be a breathing pattern selected from a number of breathing patterns that are pre-stored in the processing device or that are retrieved e.g. wirelessly or via a cable from a storage unit located distantly from the measurement device. Thus, the processing device can be configured to detect the presence of one or more breathing patterns in the subject based on the measured temperature data and preferably also based on one or more breathing patterns that are pre-stored in the processing device or that are retrieved e.g. wirelessly or via a cable from a storage unit located distantly from the measurement device. The processing device is in this case advantageously configured to compare and particularly correlate the measured temperature data with/to the pre-stored breathing patterns, in order to indicate, whether one of these breathing patterns applies for the measured subject. The correlation can be carried out by means of known statistical methods or by applying artificial intelligence.

Additionally or in other embodiments, the at least one vital value can also concern the core temperature and/or the cardiac function of the subject. With regard to the cardiac function, the processing device can for example be configured to determine the number of heartbeats per minute based on the temperature data measured on the two spatially different skin regions. It has been found, in this respect, that the pulsation of the heart is associated with small temperature changes that can be measured on the skin surface.

The one or several thermal sensors can be provided such, that each sensor is directed to one region of the skin surface of the subject and that, thus, each one of the spatially different regions is measured by a different thermal sensor, i.e. each thermal sensor is associated to one specific region. Preferred, however, is an embodiment with not more than two thermal sensors, more preferred with only a single thermal sensor. In this case, the single sensor or at least one of the two thermal sensors is preferably a thermal camera that covers a plurality of anatomical regions of the subject or even covers the entire subject. In such an embodiment, the processing device can particularly be configured to filter the measured temperature data, which in this case preferably are in the form of one or several thermal images, such that temperature data associated with spatially different regions are used for the determination of the at least one vital value. It is thus possible that the measurement device comprises a single thermal sensor only.

The one or several thermal sensors are preferably devices that use the infrared radiation of the measured subject to output the temperature data. For this purpose, the one or several thermal sensors are preferably sensitive to wavelengths from about 1'000 nm to about 14'000 nm, more preferably from about 9'000 nm to about 14'000 nm.

For measuring the temperature data of the subject, the one or several thermal sensors do not have to be in contact with the skin of the subject. Thus, the measurement is carried out with the one or several thermal sensors being located distantly from the skin surface of the measured subject. Preferably, the entire measurement device is adapted to measure the temperature data and to determine the at least vital value while being located at a distance from the measured subject. In this way, the measurement is more comfortable for the subject and the measured data are less influenced by the measurement situation.

The at least two spatially different regions can be regions of differing anatomical body regions of the subject. For example, a first region can be in the face of the subject and a second, spatially different region can be a hand of the subject. Particularly preferred, however, is an embodiment in which the at least two spatially different regions are differing anatomical regions of the face of the measured subject. For example, a first region can be the region between the nose and the upper lip and a second region can be a cheek or the forehead of the measured subject. The at least two spatially different regions are preferably spatially completely delimited from each other, i.e. do not overlap. The two spatially different regions can be directly adjacent to each other or be located at a distance from each other.

A measurement directly on the skin surface of the subject means that the measured temperature data reflect the temperature of the skin surface of the respective region. Thus, the temperature of the skin surface is measured rather than the temperature of the air that surrounds the measured subject.

Preferably at least one of the at least two spatially different regions is located in the nose-mouth area of the subject and the skin temperature of a location is measured and used for the determination of the at least one vital value, which is particularly affected by the health status of the subject. The nose-mouth area concerns the area of the body, where the influence of the inhale and exhale breath on the skin temperature is maximal and, thus, where the skin temperature is mostly affected by the breathing behaviour. The at least one region preferably covers not more than the nose-mouth area. It can particularly be limited on one or a plurality of the areas including the area of the nostrils, the tip of the nose, the upper and/or lower bridge of the nose, the area between the nose and the upper lip and the area of the lips. At least one further region of the at least two spatially different regions can also be located in the nose-mouth area and be likewise limited on one or a plurality of the areas including the area of the nostrils, the tip of the nose, the upper and/or lower bridge of the nose, the area between the nose and the upper lip and the area of the lips.

The processing device preferably comprises at least a control unit, e.g. in the form of a printed circuit board (PCB), and/or a storage element. The determination of the at least one vital value on the basis of a difference in the measured temperature is preferably preprogrammed in the processing device, e.g. on the PCB.

The at least one vital value can for example be determined by means of artificial intelligence. For this purpose, the processing device can comprise a neural network. The neural network can be in the form of executable software code stored in a storage element of the processing device, or it can be hard-wired in the electronics of the processing device. It is, however, also possible that the at least one vital value is determined for example by a mathematical calculation applied to the measured temperature data or by an extraction of certain values of the measured temperature data, such as for example maximum, minimum or average values, or by a combination of a mathematical calculation, an extraction of certain values or a similar algorithm.

The processing device is preferably configured to determine the at least one vital value such, that in a first step a difference between the measured temperature data associated to the spatially different regions of the subject is calculated, which in a second step is then used to determine, e.g. by carrying out certain pre-defined mathematical calculations, the at least one vital value.

In a particularly preferred embodiment, the vital value is a measure for the quality of respiration and the processing device is configured to determine the vital value on the basis of a spatial change, in particular on the basis of a spatial and temporal change, in the temperature data measured in the nose-mouth area of the subject. It has surprisingly been found, in this respect, that the spatial distribution of the skin surface temperature in the nose-mouth area during certain time points of the breathing cycle and particularly the change over time of this spatial distribution in the nose-mouth area over the breathing cycle is dependent on the breathing quality. In the case of a congestion of the airways or in the presence of certain heath issues, the spatial temperature distribution in the nose-mouth area and particularly the temporal change of this temperature distribution during the breathing cycle are often changed, what can be used by the measurement device in the determination of the at least one vital value. These temperatures changes are most detectable in the area directly below the nostrils and on the nostrils from both front and side views and are in some cases better detectable during the inhale-phase and in some cases in the exhale-phase. Thus, at least one of the at least two regions is preferably limited on the area of the tip of the nose and in particular on the area immediately around the nostrils. The processing device is in this case advantageously configured to detect the inhale- or the exhale-phase of the respiration cycle and to determine the at least one vital value based on the temperature data measured during the respectively detected inhale- or exhale-phase only.

It has also been found, that in thermal images of the nose, the temperature change transits up the length of the nose, i.e. from the tip to the root of the nose, during inhale and down the nose during exhale. The extent of spatial change of temperature can be used as an indicator for the amplitude of respiration. Consequently, the processing device can particularly be configured to assess the course of the temperature change along the length of the nose during a respiration cycle. The at least two spatially different regions can then be represented for example in a function determined by the processing device, which relates the measured skin temperature to the spatial location along the length of the nose, preferably for several time points during the respiration cycle. This allows a determination of vital values e.g. in the form of the breathing rate, of the relative speed of the inhale- and exhale-phase (sinus curve shape extrapolation) and/or of the amplitude (corollary to volume) of the inhaled or exhaled breath.

The measurement device can be configured to regularly or continuously monitor the spatial change of the temperature around the nose and mouth, in order to determine the breathing quality and airflow.

In another particularly preferred embodiment, the vital value can be an estimate for the core temperature of the subject. The measurement device in this case preferably comprises an additional further thermal sensor which is coupled to the processing device and serves for the measurement of the ambient temperature. The processing device of this embodiment is preferably configured to combine the temperature data measured in the at least two spatially different regions by means of the one or several thermal sensors with the ambient temperature measured by means of the further thermal sensor, in order to determine the vital value. Thus, for the determination of the core temperature of the subject, the processing device is preferably configured to combine the temperature measurements of the at least two spatially different skin regions with the measurement of the ambient temperature. This is based on the finding that the core temperature is determined more reliably, if not only the temperature as measured in the nose-mouth area is considered. A much more reliable determination of the core temperature can be achieved, if the processing device is configured to consider not only the nose-mouth temperature, but in addition the temperature at a spatially different region of the subject, such as e.g. the temperature at a cheek, hand or forehead, as well as the ambient temperature. The processing device is preferably configured to calculate the core temperature based on a mathematical formula that considers the temperature of the nose-mouth region, the temperature at a spatially different region and the ambient temperature. The temperature of the nose-mouth region can be limited on the temperature as measured on one or a plurality of the areas including the area of the nose, the area of the nostrils, the tip of the nose, the upper and/or lower bridge of the nose, the area between the nose and the upper lip and the area of the lips.

Preferably, at least one of the one or several thermal sensors is a thermal camera. The thermal camera is preferably adapted to cover more than a single anatomical face region, more preferably more than a single anatomical body region and most preferably the entire subject. The thermal camera is advantageously adapted to measure temperature data in the at least two spatially different regions. The thermal camera can be the only thermal sensor of the measurement device.

As already mentioned, preferably at least one, more preferably at least two, of the at least two spatially different regions is/are located and advantageously limited on one or a plurality of the following areas: the nose, the tip of the nose, the upper and/or lower bridge of the nose, immediately around the nostrils, between the nose and the upper lip, the lips, the lips and the area immediately around the lips.

In a preferred embodiment, the measurement device additionally comprises an imaging device coupled to the processing device. The imaging device is preferably an RGB and/or a monochrome and/or a near-infrared imaging device, in particular a CMOS- or CCD-sensor, more particularly a CMOS- or CCD-camera. A time-of-flight camera can basically also be used a the imaging device. The imaging device can be adapted to operate in the visible spectrum or in other spectra, such as e.g. the infrared-spectrum. The provision of an imaging device allows the recognition of characteristic points of interest in a face (e.g. eyes, mouth, nose), in order to determine a spatial region-of-interest for thermal imaging or to filter out one or several regions-of-interest with respect to the measured temperature data. Thus, the processing device of such an embodiment is preferably configured to identify one or several regions-of-interest based on the data acquired by the imaging device and to filter out measured temperature data that are associated to this/these region(s)-of-interest, in order to determine the at least one vital value based on the filtered temperature data.

In a particularly preferred embodiment, the processing device is configured to identify a single human or animal subject in the image data received from the imaging device. Thus, the imaging device of this embodiment is preferably adapted to capture a scene with possibly a plurality of subjects, and the processing device is configured to identify a single subject or to separate out the subjects, in order to then determine the at least one vital valued for the identified subject or subjects.

It is also preferred that the processing device is configured to identify a certain region-of-interest (ROI) or a plurality of ROI's for the subject, in particular for the previously identified subject, based on the image data received from the imaging device. The ROI('s) to be identified can particularly be located in the nose-mouth area of the subject.

The processing device can be configured to identify the inhale- and/or exhalephase of the subject and to determine the at least one vital value, in particular if it is a measure for the quality of respiration, on the basis of temperature data measured during the inhale- and/or exhale-phase. It has been found that temperature changes in the nose-mouth area are in some cases better detectable during the inhale phase and in other cases during the exhale phase.

The processing device can particularly be configured to identify a possible congestion of the airways and/or illness of the subject based on the measured temperature data. If the processing device is configured to identify a possible congestion and/or illness directly based on the measured temperature data, the information, whether a congestion and/or illness is present can be regarded as the at least one vital value. It is also possible, however, that the processing device is configured to first determine the at least one vital value, which can for example be related to the breathing behaviour and/or to the core temperature, and to then determine the presence of a possible congestion and/or illness based on the at least one vital value.

For identifying a possible congestion of the airways and/or illness of the subject, the temperature data can also be measured at regular intervals, e.g. daily or weekly, in order to assess the development of the at least one vital value for the same subject over time. By means of such a monitoring of the at least one vital value, identification of certain illnesses such as in particular infections becomes possible.

The present invention also relates to a first method for assessing vital data of a human or animal subject, in particular by means of a first measurement device as indicated above. The method comprises at least the steps of:
- measuring, without contact, by means of one or several thermal sensors, in particular one or several thermal cameras, temperature data in at least two spatially different regions of the subject, preferably directly on the skin surface of the subject, wherein at least one of the at least two spatially different regions is located in the nose-mouth area of the subject, and
- determining at least one vital value, which is characteristic of the subject, on the basis of the temperature data measured in the at least two spatially different regions.

A further object of the present invention is to improve the reliability of the assessment of vital data by means of radar-measurements.

This object is solved by a second type of measurement device as claimed in claim 16 and by a second method as claimed in claim 18. Further embodiments of the measurement device and of the method are provided in the dependent claims.

The present invention thus also provides a second type of measurement device for assessing vital data of a human or animal subject, wherein the second type of measurement device can be, but does not need to be, designed as the first type of measurement device explained above, i.e. combine the determination of at least one vital value based on a contactless measurement of temperature data with the determination of the same or a further at least one vital value by means of radar measurement. The second type of measurement device, i.e. the measurement device for determining at least one vital value by means of radar measurement comprises
a CMOS-sensor and/or a time-of-flight(ToF)-sensor, in particular a CMOS-camera and/or a time-of-flight(ToF)-camera, for obtaining measurement data from the subject without contact;
a radar emitter for emitting a radar wave;
a radar receiver adapted to receive the radar wave after it has been reflected and/or transmitted by the subject; and
a processing device which is coupled to the CMOS-sensor and/or to the time-of-flight(ToF)-sensor and to the radar receiver;
wherein the processing device is configured to identify a region-of-interest based on the measurement data received from the CMOS-sensor and/or from the time-of-flight(ToF)-sensor and is further configured to determine at least one vital value, which is characteristic of the subject, on the basis of data received from the radar receiver with respect to the identified region-of-interest.

Thus, the processing device is configured to combine the measurement data received from the CMOS- and/or ToF-sensor with the one received from the radar emitter/receiver, in order to improve the assessment of the vital data, in particular with regard to the reliability of the data. By means of the CMOS- and/or ToF-sensor, image data of the measured scene can be obtained with a high spatial resolution as concerns azimuth and elevation as well as, in the case of a ToF-sensor, depth. These image data are preferably used by the processing device for the identification of a single subject and/or for the identification of a region-of-interest (ROI) within the subject. The ROI can for example be the chest or the face of a person or even smaller anatomical regions, such as the nose-mouth area, the eyes, a cheek, the forehead, the lips, the nose and/or the region between the nose and the upper lip.

The data received from the radar receiver can then be e.g. filtered, such that only signal components received from the radar are used for the determination of the at least one vital value, which are associated to this identified ROI.

The at least one vital value can for example be determined by means of artificial intelligence. For this purpose, the processing device can comprise a neural network. The neural network can be in the form of executable software code stored in a storage element of the processing device, or it can be hard-wired in the electronics of the processing device.

The radar emitter/receiver usually has a high accuracy and high spatial and temporal resolution with respect to the measurement of depths. However, radar emitters/receivers usually have a relatively low spatial resolution as concerns azimuth and elevation. By combining the data received from the radar emitter/receiver with the high resolution image data of the CMOS- and/or ToF-sensor, this drawback of the radar emitter/receiver can be overcome. In other words, by combining multiple sensors with different principles in a single multiple-sensor device, the difficulty to e.g. track a spot of interest can be overcome.

The radar emitter/receiver can have multiple transmitting and receiving antennas (MIMO), in order to improve the spatial resolution of azimuth and/or elevation with regard to the distance measurement.

With a second type of measurement device as indicated above, it is possible to determine the vital signals of a single subject even if there is a group of subjects in the detection zone of the sensors. In addition, the device can be adapted to filter out temporal variations of the location of the region of interest that are larger than the amplitude of the depth signal received from the radar receiver - also known as gross motion - by subtracting the position difference from the signal.

To overcome problems with changing light conditions both the first type and the second type of measurement device system can additionally comprise an illumination element for actively illuminate the measured scene. The illumination element is preferably adapted to illuminate the measured scene by means of infrared light, which has the advantage to be invisible for the human and/or animal subjects in the scene.

Possible deviations of the field-of-views between the sensors and the radar emitter/receiver are preferably measured and stored in the measurement device during production. The processing device can then be configured to consider such deviations in the signal processing, i.e. in the determination of the at least one vital value based on the sensor signals.

The radar emitter and the radar receiver together form a radar-based sensing device which is preferably adapted to emit radar waves in a frequency range from 15 GHz to 100 GHz, in particular from 18 GHz to 27 GHz or from 40 GHz to 110 GHz. The use of frequencies in these ranges leads to wavelengths in the millimetre or low centimetre range, meaning that a sufficient spatial resolution for the depth measurement is achieved in most applications. Furthermore, in this frequency range, a major part of the radar waves is reflected by the measured subject(s), such that the required measurement and/or detection becomes feasible in practise. Radar emitters/receivers that are working in the frequency range as indicated above are also known as mmWave radar sensors.

In a particularly preferred embodiment, the radar-based sensing device is a frequency-modulated continuous-wave (FMCW) radar system. An FMCW radar system is particularly well suited to also measure velocities and/or accelerations. Thus, an FMCW radar system is particularly well suited for the assessment of vital data based on the measurement of spatial changes of the skin surface of humans or animals. The measurement device can particularly be adapted to assess spatial changes of the skin surface that are due to the respiration and/or due to cardiac motion, in order to determine the at least one vital value.

The present invention also relates to a second method for assessing vital data of a human or animal subject, in particular by means of a second measurement device as indicated above. The method comprises at least the steps of:
- obtaining data from the subject by means of a CMOS-sensor and/or a time-of-flight(ToF)-sensor;
- emitting a radar wave by means of a radar emitter;
- receiving, by means of a radar receiver, the radar wave which has been reflected and/or transmitted by the subject;

- identifying a region-of-interest based on the data obtained from the CMOS-sensor and/or from the time-of-flight(ToF)-sensor;
- filtering an output signal obtained from the radar receiver such that a filtered signal is obtained which is limited, i.e. associated only, to the identified region-of-interest; and
- determining at least one vital value, which is characteristic of the subject (35), on the basis of the filtered signal.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: schematically shows a human face with possible regions-of-interest where measurable temperature changes due to respiration can occur;
- Fig. 2: schematically shows the human face of Fig. 1 with a segmentation grid as applied by the processing device of a measurement device according to the invention, in order to identify certain regions-of-interest;
- Fig. 3: shows examples of the time course of normal and abnormal breathing patterns;
- Fig. 4: schematically shows the functional components of a first type of measurement device according to an inventive embodiment;
- Fig. 5: shows a flow chart of a method as applied by the measurement device of Fig. 4 for determining the breathing pattern by means of a thermal camera;
- Fig. 6: shows a group of human subjects measured by the measurement device of Fig. 4;
- Fig. 7: shows the identification of single subjects by means of the measurement device of Fig. 4;
- Fig. 8: schematically shows the functional components of a second type of measurement device according to an inventive embodiment; and
- Fig. 9: shows the identification of a single subject out of a group of human subjects and the further identification of a region-of-interest on the single subject by means of the measurement device of Fig. 8.

### DESCRIPTION OF PREFERRED EMBODIMENTS

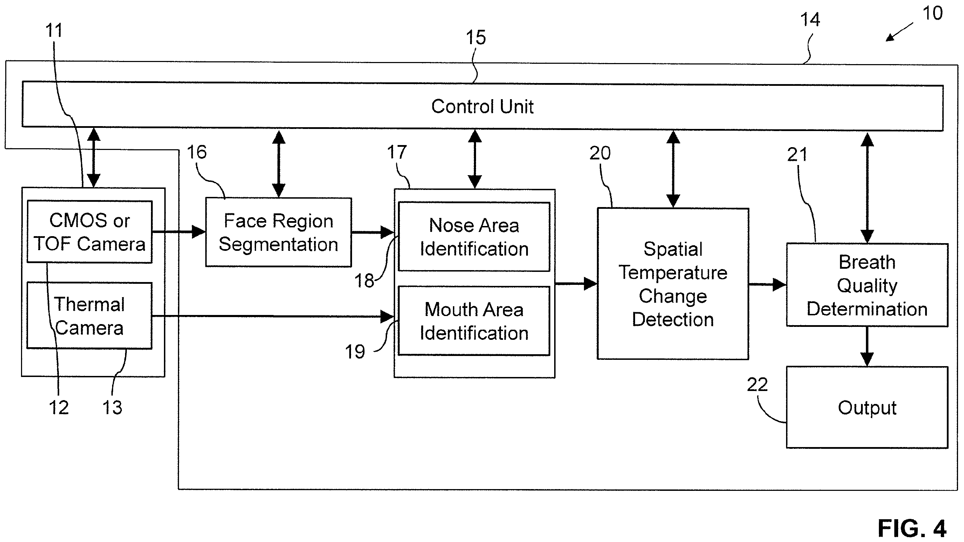

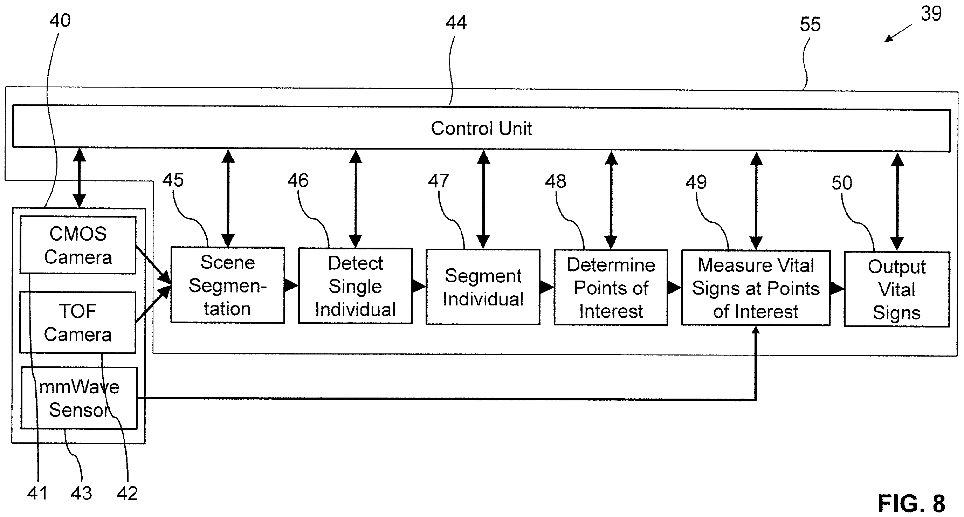

In the figures, preferred embodiments of inventive measurement devices and methods are shown, which allow the assessment of vital data of a human or an animal subject. Particularly, Figure 4 schematically shows the functional components of a first type of measurement device and Figure 8 shows the functional components of a second type of measurement device. Elements or components that have the same or a similar function are annotated by the same reference numerals in the figures, even if they belong to different embodiments or different types of measurement devices.

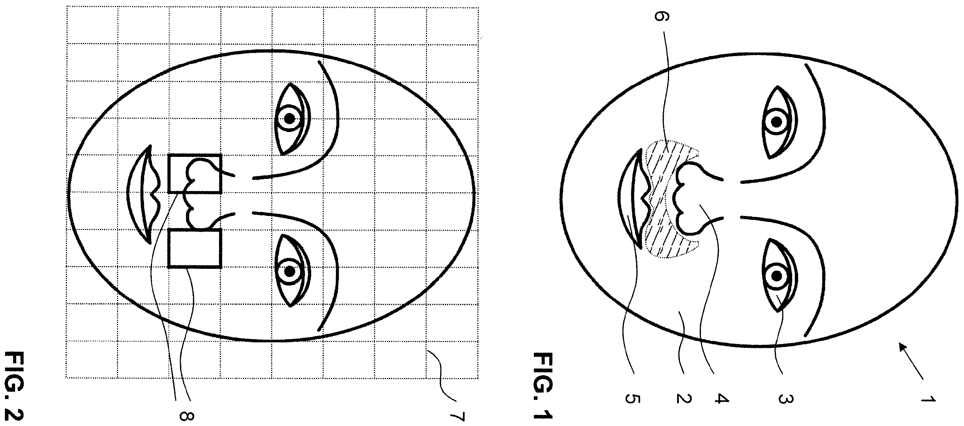

Figure 1 shows the face 2 of a human subject 1. The exposed skin surface of the face 2 allows a contactless measurement of temperature, in order to assess a vital value or a plurality of vital values of the subject 1. The vital value can particularly relate to the core temperature or to the breathing behavior of the subject 1, which are both reflected in spatial temperature changes on the skin surface of the face 2. Possible and preferred regions-of-interest 6, where such spatial temperature changes can be detected, are shown in figure 1. These regions-of-interest 6 are located in the nose-mouth-area and particularly in the area between the nose 4 and the lips 5 and around the tip of the nose 4. Thus, for assessing e.g. the core temperature and/or the breathing behavior of the subject 1, the skin temperature of at least two spatially different regions-of-interest 6 can be measured and set in relation to each other by means of a processing device. In particular, differences in the measured temperature data between the spatially different regions-of-interest 6 can be detected and used for determining the at least one vital value.

The measurement of the skin temperature is preferably carried out by means of one or several thermal sensors, more preferably by a thermal camera. The thermal camera can cover and image the entire face 2 or even the entire subject 1. The regions-of-interest 6, which are used for the determination of the at least one vital value, can be identified by means of a further sensor, such as an imaging device in the form of e.g. a RGB imaging device. The image(s) captured by the imaging device, can be further processed by the processing device, in order to identify the regions-of-interest 6 and to determine the at least one vital value based on the temperature data measured by the thermal camera in the respective regions-of-interest 6. This is preferably achieved by spatially filtering the temperature data measured by the thermal camera based on the previous identification of the regions-of-interest 6 in the image(s) captured by the imaging device.

In order to identify the required regions-of-interest 6 in the image(s) captured by the imaging device, the processing device is preferably configured identify the face 2 of the subject 1 in the captured image(s) and to then virtually overlay the face 2 with a segmentation grid 7, as shown in Figure 2. The identification of the face 2 is preferably carried out on the basis of landmark points, such as the eyes 3, the nose 4 and the lips 5. Within the segmentation grid 8, the desired regions-of-interest 8 in the nose-mouth-area of the face 2 can then be identified in the form of respectively selected segments of the segmentation grid 7.

If the at least one vital value that is assessed by the measurement device is the core temperature of the subject 1, a first region-of-interest is preferably located in the nose-mouth-area of the face 2 and a second region-of-interest is preferably located outside the nose-mouth-area. Particularly, the first region-of-interest can be in the area between the nose 4 and the lips 5 and/or the second region-of-interest can be located on one or both of the cheeks or on the forehead of the subject 1. The determination of the core temperature of the subject 1 is then preferably based on the temperature data measured in the first region-of-interest, i.e. the nose-mouth-area, as well as on the difference in the measured temperature data between the first and the second regions-of-interest. For this purpose, the temperature difference between these spatially different regions is analyzed by the processing device for example by means input variable of a mathematical formula and/or as an input of an artificial intelligence computing unit, e.g. a neural network.

If the at least one vital value relates to the core temperature of the subject 1, the measurement device preferably comprises a further thermal sensor for detecting the ambient temperature in the region of the subject 1. The measured ambient temperature is advantageously also taken into account in the determination of the core temperature based on the measured temperature data. Thus, for determining the core temperature of the subject 1, the processing device of the measurement device is preferably configured to take into account the temperature data measured in the first region-of-interest, i.e. the nose-mouth-area, as well as the difference in the measured temperature data between the first and the second regions-of-interest and the measured ambient temperature. The measured ambient temperature is preferably also used as an input variable for the mathematical formula and/or the neural network as applied or provided by the processing device. By using the skin temperature of the nose-mouth-area, the difference in the temperatures between a region-of-interest in the nose-mouth-area and a region-of-interest outside of the nose-mouth-area as well as the ambient temperature, a particularly accurate and reliable determination of the core temperature of the subject 1 can be achieved by the measurement device.

Alternatively or additionally, the at least one vital value can also relate to the breathing behavior of the subject 1. For this purpose, the temperature difference between a first region-of-interest in the nose-mouth area and a second region-of-interest outside the nose mouth area is preferably taken into account by the processing device and advantageously combined with a temperature measured in the nose-mouth area. The at least one vital value can in this case for example be the breathing volume, the breathing rate, the inhale or exhale volume and/or an entire time course of the breathing process during one or several respiration cycles. The at least one vita value can also be used for or be represented by an indicator for a certain breathing pattern. The identification of a certain breathing pattern can be a direct indicator for a possible congestion of the airways or an illness, such as a virus-infection, of the subject 1.

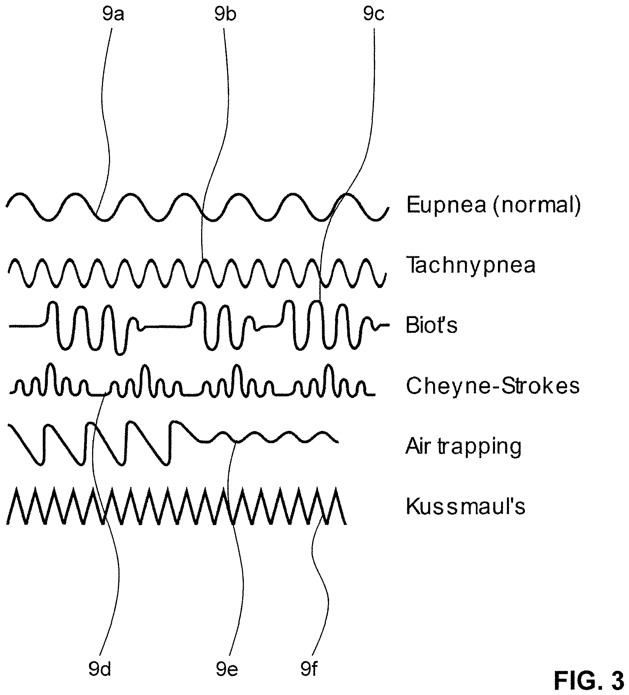

In order to identify a breathing pattern based on the measured temperature data, a number of possible breathing patterns can be pre-stored in the processing device. The processing device can be configured to compare the measured temperature data with the pre-stored breathing patterns, in order to find a match. The breathing patterns can for example be pre-stored in the form of a lookup-table in a storage element of the processing device. The comparison can for example be carried out by means of artificial intelligence, in particular by means of an artificial neural network. A possible set of breathing patterns 9a-9f is shown in Figure 3: Breathing pattern 9a relates to Eupnea, i.e. normal breathing. Breathing pattern 9b represents Tachypea, pattern 9c Biot's respiration, pattern 9d Cheyne-Stokes respiration, pattern 9e air trapping and pattern 9f Kussmaul's respiration.

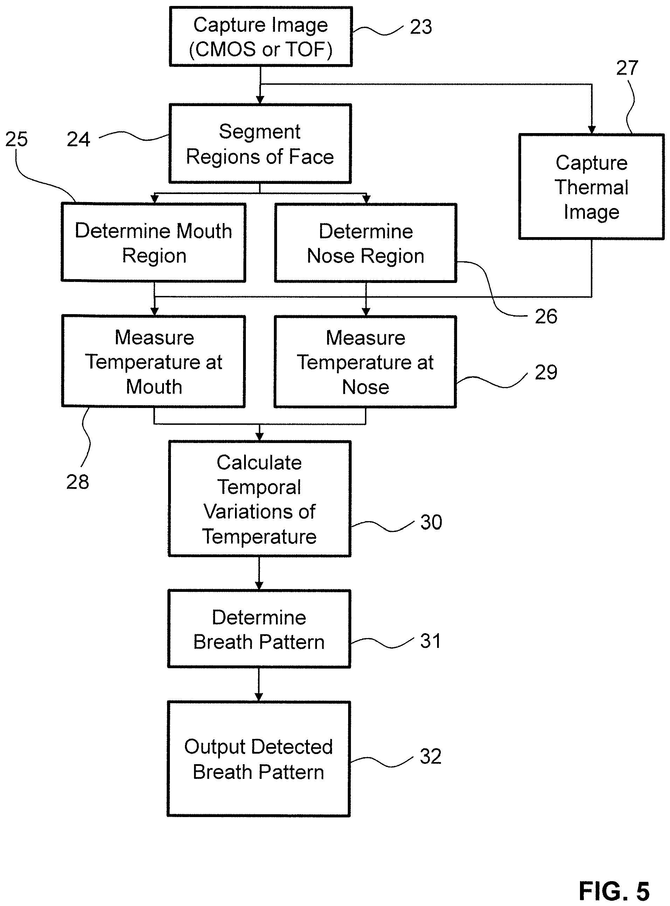

Figure 4 schematically shows an inventive embodiment of a first type of measurement device 10 for assessing the breathing quality of a human or animal subject. A flow chart which illustrates the method steps as applied by the measurement device 10 is shown in Figure 5.

The measurement device 10 as shown in Figure 4 comprises a multiple sensor module 11 with an image device in the form of a CMOS- or time-of-flight(ToF)-camera 12 and with a thermal camera 13. The multiple sensor module 11 is coupled to a processing device 14 of the measurement device 10. The processing device 14 is adapted to control the multiple sensor module 11 and to post-process the measurement data received from the multiple sensor module 11, in order to output an indication with regard to the breathing quality of the subject 1. For this purposed, the processing device 14 comprises a control unit 15 which is in communication with the multiple sensor module 11 as well as with several functional components of the processing device 14. The control unit 15 particularly serves to control the multiple sensor module 11 as well as the data flow within the processing device 14.

The image data captured by the CMOS- or ToF-camera 12 in method step 23 of Figure 5 are transmitted to a face region segmentation unit 16 of the processing device 14. The face region segmentation unit 16 is adapted to identify the region of the face 2 of the imaged subject 1 based on the image(s) captured by the CMOS- or ToF-camera 12 (method step 24 in Figure 5), as has been described further above with respect to Figure 2.

Together with the temperature data measured by the thermal camera 13, the output of the face region segmentation unit 16 is fed to a nose-mouth area identification unit 17 of the processing device 14. In the nose-mouth area identification unit 17, at least one region-of-interest in the nose-mouth area is identified with the help of e.g. on a segmentation grid 7, as explained further above with respect to Figure 2. In the present embodiment, the face region segmentation unit 16 is configured to identify both the nose area, i.e. the area of the nose 4 (method step 26), and the mouth area, i.e. the area of the lips 5 (method step 25). For this purpose, the face region segmentation unit 16 comprises a nose area identification unit 18 and a mouth area identification unit 19. The outputs of the nose area identification unit 18 and the mouth area identification unit 19 are used by the face region segmentation unit 16 to spatially filter the temperature data of the thermal camera 13 such that only temperature data associated with the nose area and the mouth area are considered further. Instead of filtering the temperature data of the thermal camera 13 with respect to the nose- and mouth-areas, it would also be possible to specifically measure the temperatures in the nose-area (method step 29) and in the mouth-area (28) based on the outputs of the nose area identification unit 18 and the mouth area identification unit 19. The output of the face region segmentation unit 16 can for example be in the form of two data streams, one comprising temperature data of the nose area and the other temperature data of the mouth area. The temperature data are preferably spatially averaged by the face region segmentation unit 16 for each area, i.e. the nose area and the mouth area.

The output of the face region segmentation unit 16 is provided to a spatial temperature change detection unit 20, which also represents a functional component of the processing device 14. In the spatial temperature change detection unit 20, the temperature data as measured for the nose area and for the mouth area are analyzed. In this analysis, spatial temperature changes between the nose area and the mouth area e.g. during the inhale- or exhale-phase are detected and quantified over time, i.e. for example over one or several respiration cycles (method step 30 in Figure 5).

The detected temperature changes are fed from the spatial temperature change detection unit 20 to a breath quality determination unit 21, in which an indication concerning the breath quality of the measured subject 1 is determined based on the detected temperature changes. The indication can for example be in the form of a selection of one of several pre-stored breathing patterns (method step 31), as the breathing patterns 9a-f shown in Figure 3. The output of the breath quality determination unit 21 is fed to an output unit 22 which indicates an output with respect to the breathing quality of the subject 1 to a user, which can be for example the medical personnel or the subject 1 himself (method step 32). An output device can be provided such as for example a display and/or a sound device, e.g. an alarm.

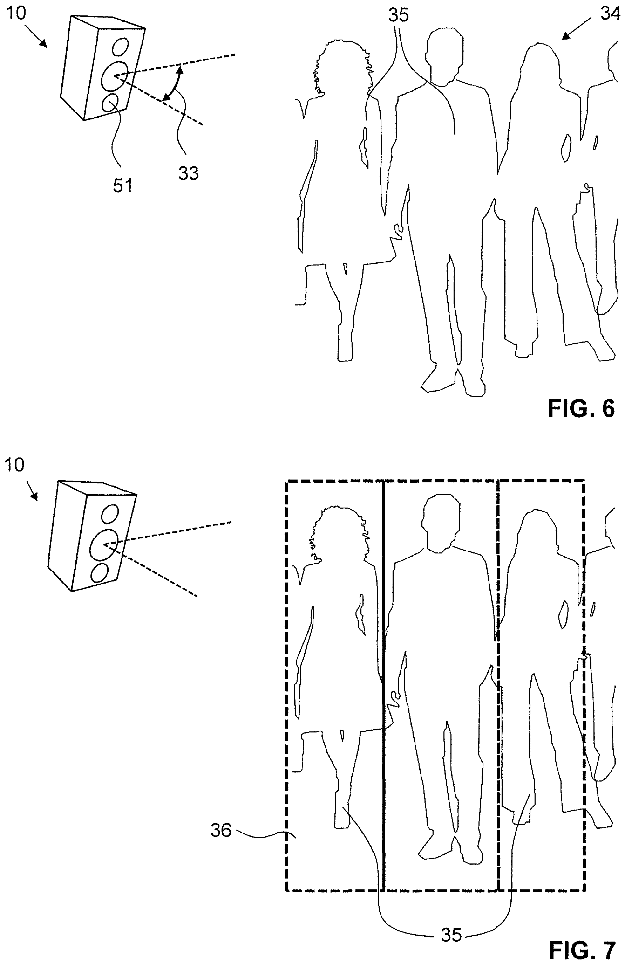

Figure 6 shows a scene 34 with a group of subjects 35 which is captured by an inventive measurement device 10. The measurement device 10 can particularly be designed in accordance with the measurement device 10 as shown in Figure 4. The thermal camera and the imaging device of the measurement device 10 are contained in a common housing together with the processing device 14 (not shown in Figure 6) and are capturing data within a measuring range 33. The measuring range 33 is wide enough that a plurality of single subjects 35 can be imaged and measured at the same time.

In order to assess the one or several vital values of a subject, it is necessary to first identify a single subject 35 in the captured scene 34, in order to then identify the regions-of-interest 8 as explained further above with regard to Figures 2, 4 and 5. For identifying the single subjects 35, the captured image of the scene 34 can be segmented and overlaid by a segmentation grid 36 as shown in Figure 7. The vital data can then be assessed for some or all of the identified single subjects 35 in accordance with the method as shown in Figure 5. The measurement device 10 of Figures 6 and 7 also has an ambient temperature sensor 51, in order to e.g. not only assess the breathing quality of the subjects 35, but also the core temperature.

Figure 8 schematically shows an inventive embodiment of a second type of measurement device 39 for assessing vital data of a human or animal subject.

The measurement device 39 comprises a multiple sensor module 40 with a CMOS-camera 41, a ToF-camera 42 and a mmWave-sensor 43. The mmWave-sensor 43, which is a radar sensor with a radar emitter and a radar receiver, serves to measure the movement of the skin of an animal or human subject, by sending out and receiving radar waves that are reflected and/or transmitted by the subject. The mmWave-sensor 43 is able to precisely measure with high temporal and spatial resolution as concerns depths, but with relatively low spatial resolution as concerns elevation and azimuth. The CMOS-camera 41 and the ToF-camera 42, on the other hand, are able to measure with relatively high spatial resolution as concerns elevation and azimuth. The CMOS-camera 41, however, is not able to measure depth. The ToF-camera 42 is able to measure depth, but with a limited spatial resolution when compared to the mmWave-sensor 43. In the measurement device 39 the advantages of the CMOS-camera 41, the ToF-camera 42 and the mmWave-sensor 43 are combined to measure depths with high temporal and spatial resolution in all dimensions.

The measurement device 39 also comprises a processing device 55 with a plurality of function components. One of these components is a control unit 44, which serves for controlling the multiple sensor module 40 as well as the data flow within the processing device 55.

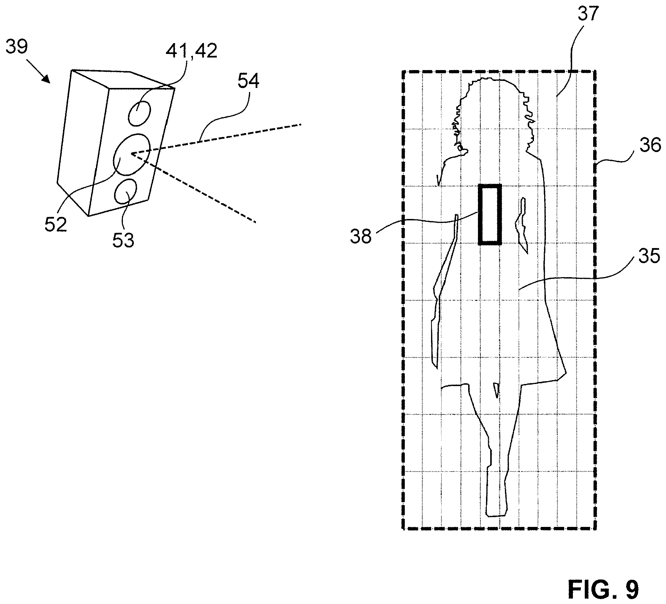

The image data captured by the CMOS-camera 41 and the ToF-camera 42 are provided to a scene segmentation unit 45, which segments the scene captured by the CMOS-camera 41 and the ToF-camera 42. Similarly as explained above with respect to Figure 7, a segmentation grid 36 can be used for this purpose (see also Figure 9). Single individual subjects 35 are then detected and identified (detect single individual 46).

After the single subjects 35 have been identified, each or at least one of the subjects 35 is individually segmented. For this purpose, a subsegmentation grid 37 can be applied by the processing device 55 for segmenting the single subject into a plurality of subsegments. One or several of these subsegments can be chosen as a region-of-interest 38 (see Figure 9). For example, as shown in Figure 9, the chest of the subject 35 can be chosen as the region-of-interest 38 by means of an image recognition algorithm, which can for example be based artificial intelligence. Instead of a region-of-interest, it is also possible to select a particular point-of-interest. This method step is carried out by a respective functional component of the processing device 55 ("segment individual" 47 and "determine points-of-interest" 48 in Figure 8).

The identified region-of-interest 38 is then used to specifically measure the vital data in the respective region or point(s) of the subject 35 by means of the mmWave-sensor 43 or to filter the data measured by the mmWave-sensor 43 such, that only data associated to the respective region-of-interest 38 or point(s)-of-interest are further considered in the assessment ("measure vital signs at points-of-interest" 49).

The measurement data acquired by the mmWave-sensor 43 in the respective region(s)- or point(s)-of-interest is then used to determine at least one vital value, which is characteristic of the subject. The at least one vital value can for example be the heart rate or the breathing rate of the subject 35. I could be shown, that motions on the skin surface and even on cloth surfaces due to breathing and due to cardiac movements can be detected with an mmWave-sensor 43. The determined value(s) are output via a display or sound device ("output vital signs" 50).

Figure 9 shows a possible embodiment of the measurement device 39 with a CMOS- and/or the ToF-camera 41, 42 and with a mmWave-sensor comprising a radar emitter 52 and a radar receiver 53 which are contained in a single housing together with the processing device 55 (not visible in Figure 9). A radar wave 54 which is emitted by the radar emitter 52 is reflected and/or transmitted by the subjects 35 before being received by the radar receiver 53. The measurement device 39 can for example be used to analyze the chest movement of the subject 35, e.g. with respect to cardiac and/or respiratory motions.

**LIST OF REFERENCE SIGNS**

| | | | |
|---|---|---|---|
| 1 | Human subject | 11 | Multiple sensor module |
| 2 | Face | 12 | CMOS- or ToF-camera |
| 3 | Eyes | 13 | Thermal camera |
| 4 | Nose | 14 | Processing device |
| 5 | Lips | 15 | Control unit |
| 6 | Regions-of-interest | 16 | Face region segmentation |
| 7 | Segmentation grid | | unit |
| 8 | Region-of-interest | 17 | Nose-mouth area identification unit |
| 9a-f | Breathing patterns | 18 | Nose area identification unit |
| | | 19 | Mouth area identification unit |
| 10 | Measurement device | 20 | Spatial temperature change |
| | detection unit | 36 | Segmentation grid |
| 21 | Breath quality determination unit | 37 | Subsegmentation grid |
| | | 38 | Region-of-interest |
| 22 | Output unit | 39 | Measurement device |
| 23 | Capture image (CMOS or | 40 | Multiple sensor module |
| | ToF) | 41 | CMOS-camera |
| 24 | Segment regions of face | 42 | ToF-camera |
| 25 | Determine mouth region | 43 | mmWave-sensor |
| 26 | Determine nose region | 44 | Control unit |
| 27 | Capture thermal image | 45 | Scene segmentation |
| 28 | Measure temperature at mouth | 46 | Detect single individual |
| | | 47 | Segment individual |
| 29 | Measure temperature at nose | 48 | Determine points-of-interest |
| 30 | Calculate temporal variations of temperature | 49 | Measure vital signs at points-of-interest |
| 31 | Determine breath pattern | 50 | Output vital signs |
| 32 | Output detected breath pattern | | |
| | | 51 | Ambient temperature sensor |
| | | 52 | Radar emitter |
| 33 | Measuring range | 53 | Radar receiver |
| 34 | Scene | 54 | Radar wave |
| 35 | Single subject | 55 | Processing device |

## Claims

1. A measurement device (10) for assessing vital data of a human or animal subject (1, 35), comprising:
one or several thermal sensors (13) for the contactless measurement of temperature data in at least two spatially different regions (6, 8) of the subject (1, 35), wherein at least one of the at least two spatially different regions (6, 8) is located in the nose-mouth area of the subject (1, 35); and
a processing device (14) which is coupled to the one or several thermal sensors (13) and which is configured to determine at least one vital value, which is characteristic of the subject (1, 35), on the basis of a difference in the measured temperature data between the spatially different regions (6, 8) of the subject (1, 35).

2. The measurement device (10) of claim 1, wherein the vital value is a measure for the quality of respiration, and wherein the processing device (14) is configured to determine the vital value on the basis of a spatial change, in particular on the basis of a spatial and temporal change, in the temperature data measured in the nose-mouth area of the subject (1, 35).

3. The measurement device (10) of claim 1 or 2, wherein the vital value is an estimate for the core temperature of the subject (1, 35), wherein the measurement device (10) additionally comprises a further thermal sensor (51) which is coupled to the processing device (14) and serves for the measurement of the ambient temperature, and wherein the processing device (14) is configured to combine the temperature data measured in the at least two spatially different regions (6, 8) by means of the one or several thermal sensors (13) with the ambient temperature measured by means of the further thermal sensor (51), in order to determine the vital value.

4. The measurement device (10) of any of the preceding claims, wherein the one or several thermal sensors (13) serve for the contactless measurement of temperature data in at least two spatially different regions (6, 8) directly on the skin surface of the subject (1, 35).

5. The measurement device (10) of any of the preceding claims, wherein at least one of the one or several thermal sensors is a thermal camera (13).

6. The measurement device (10) of any of the preceding claims, wherein at least one, preferably at least two, of the at least two spatially different regions (6, 8) is/are located on the tip of the nose (4), immediately around the nostrils, between the nose and the upper lip, on the lips (5) and/or immediately around the lips (5) of the subject.

7. The measurement device (10) of any of the preceding claims, additionally comprising an imaging device coupled to the processing device (14), the imaging device preferably being an RGB imaging device (11).

8. The measurement device (10) of claim 7, wherein the processing device (14) is configured to identify a single human or animal subject (1, 35) in the image data received from the imaging device (11).

9. The measurement device (10) of claim 7 or 8, wherein the processing device (14) is configured to identify the nose-mouth area, in particular one or a plurality of regions-of-interest (ROI) (6, 8) in the nose-mouth area, of the subject (1, 35) based on the image data received from the imaging device (11).

10. The measurement device (10) of any of the preceding claims, wherein the processing device (14) is configured to identify the inhale- and/or exhale-phase of the subject (1, 35) and to determine the at least one vital value on the basis of temperature data measured during the inhale- and/or exhale-phase.

11. The measurement device (10) of any of the preceding claims, wherein the processing device (14) is configured to determine the breathing rate and/or the amplitude of respiration on the basis of the temperature data measured in the at least two spatially different regions (6, 8) by the one or several thermal sensors (13).

12. The measurement device (10) of any of the preceding claims, wherein the processing device (14) is configured to determine whether the subject is mostly breathing through the nose (4) or through the mouth (5) based on the measured temperature data.

13. The measurement device (10) of any of the preceding claims, wherein the processing device (14) is configured to identify a possible congestion of the airways and/or illness of the subject (1, 35) based on the measured temperature data.

14. The measurement device (10) of any of the preceding claims, wherein the processing device (14) is configured to detect the presence of one or more breathing patterns (9a-f) in the subject (1, 35) based on the measured temperature data and on one or more breathing patterns (9a-f) pre-stored in the processing device (14).

15. A method for assessing vital data of a human or animal subject (1, 35), in particular by means of a measurement device (10) according to any of the preceding claims, the method comprising at least the steps of:
- measuring (27), without contact, by means of one or several thermal sensors (13), temperature data in at least two spatially different regions (6, 8) of the subject (1, 35), wherein at least one of the at least two spatially different regions (6, 8) is located in the nose-mouth area of the subject, and
- determining (31) at least one vital value, which is characteristic of the subject (1, 35), on the basis of the temperature data measured in the at least two spatially different regions (6, 8).

16. A measurement device (39) for assessing vital data of a human or animal subject (35), comprising:
a CMOS-sensor (41) and/or a time-of-flight(ToF)-sensor (42) for obtaining measurement data from the subject (35) without contact;
a radar emitter (52) for emitting a radar wave (54);
a radar receiver (53) adapted to receive the radar wave after it has been reflected and/or transmitted by the subject (35); and
a processing device (55) which is coupled to the CMOS-sensor (41) and/or to the time-of-flight(ToF)-sensor (42) and to the radar receiver;
wherein the processing device (55) is configured to identify a region-of-interest (38) based on the measurement data received from the CMOS-sensor (41) and/or from the time-of-flight(ToF)-sensor (42) and to determine at least one vital value, which is characteristic of the subject (35), on the basis of data received from the radar receiver (53) with respect to the identified region-of-interest (38).

17. The measurement device (39) of claim 16, wherein the processing device (55) is configured to identify a single human or animal subject (35) based on the data received from the CMOS-sensor (41) and/or from the time-of-flight(ToF)-sensor (42) before identifying the a region-of-interest (38).

18. A method for assessing vital data of a human or animal subject (35), in particular by means of a measurement device (39) according to any of the preceding claims, the method comprising at least the steps of:
- obtaining data from the subject (35) by means of a CMOS-sensor (41) and/or a time-of-flight(ToF)-sensor (42);
- emitting a radar wave (54) by means of a radar emitter (52);
- receiving, by means of a radar receiver (53), the radar wave which has been reflected and/or transmitted by the subject (35);
- identifying a region-of-interest (38) based on the data obtained from the CMOS-sensor (41) and/or from the time-of-flight(ToF)-sensor (42);
- filtering an output signal obtained from the radar receiver (53) such that a filtered signal is obtained which is limited to the identified region-of-interest (38);
and
- determining at least one vital value, which is characteristic of the subject (35), on the basis of the filtered signal.
